**Europäisches Patentamt**

**European Patent Office**

**Office européen des brevets**

(19)

(11) Publication number: **0 123 300**
**A2**

(12) **EUROPEAN PATENT APPLICATION**

(21) Application number: **84104488.6**

(22) Date of filing: **19.04.84**

(51) Int. Cl.³: **G 01 N 33/58**
**C 12 Q 1/00**

(30) Priority: **20.04.83 US 486924**

(43) Date of publication of application:
**31.10.84 Bulletin 84/44**

(84) Designated Contracting States:
**AT BE CH DE FR GB IT LI LU NL SE**

(71) Applicant: **ENZO BIOCHEM, INC.**
**325 Hudson Street**
**New York, N.Y. 10013(US)**

(72) Inventor: **Johnston, Kenneth H.**
**95 Horatio Street**
**New York New York 10014(US)**

(74) Representative: **Vossius Vossius Tauchner Heunemann**
**Rauh**
**Siebertstrasse 4 P.O. Box 86 07 67**
**D-8000 München 86(DE)**

(54) **Complexing of biologically active or functional compounds and methods of preparing and utilizing same.**

(57) Complexes containing a biologically active or functional compound or compounds are prepared by attaching or fixing a first compound, which may be a biologically active or functional compound, to a solid substrate under conditions such that the active site of said first compound is protected, thereupon derivatizing or combining said first compound with a derivatizing or modifying compound while said first compound is attached to said solid substrate. The resulting complex or complexed first compound is then released from attachment to said solid substrate to yield the complex, said complex comprising said derivatizing compound attached to said first compound, said first compound, upon release from the solid substrate having available its functional or biologically active site. For example, alkaline phosphatase is attached to a solid substrate, such as dextran beads, preferably through a ligand, such as a competitive inhibitor for alkaline phosphatase, e.g. para-aminobenzyl-phosphonic acid (p-ABPA). The dextran beads would be first treated with (p-ABPA) and the alkaline phosphatase brought into contact with the thus-treated dextran beads. The competitive inhibitor p-ABPA serves as a ligand for attachment of the alkaline phosphatase to the dextran beads while at the same time occupying and protecting the active site of the alkaline phosphatase. Thereupon, the thus-attached alkaline phosphatase is derivatized or modified, such as by biotinylation. Upon biotinylation of the alkaline phosphatase, the resulting biotinylated alkaline phosphatase is eluted or removed from attachment to the p-ABPA treated solid substrate or dextran beads to which it was fixed, yielding a biotinylated alkaline phosphatase with the active or functional site of the alkaline phosphatase intact. The complex, while still attached to the dextran beads, could be further treated with a second compound or component which might also, if desired, provide an active or functional site, such as by contact with avidin, which would bind to the modifying or derivatizing moiety, i.e. biotin, of the biotinylated alkaline phosphatase and the thus-produced avidin-biotinylated alkaline phosphatase complex then eluted. This complex, such as a complex comprising avidin-biotinylated alkaline phosphatase, could be employed as a detector in a detection system for the identification and-/or location of another compound, such as a biotinylated compound, e.g. a biotinylated nucleotide or biotinylated DNA.

Croydon Printing Company Ltd

Case : 19668

Enzo Biochem

VOSSIUS · VOSSIUM
HEUNEMANN
PATENT
SIEBERTSTR
TEL (089)

0123300

## COMPLEXING OF BIOLOGICALLY ACTIVE OR FUNCTIONAL

## COMPOUNDS AND METHODS OF PREPARING AND UTILIZING SAME

Enzymes have actual and great potential use as diagnostic chemicals or tools. For example, it has been proposed to biotinylate an enzyme, such as alkaline phosphatase, by attaching a biotin group thereto and to employ the resulting biotinylated enzyme as a probe, such as a diagnostic probe. Unfortunately, it has been found that when alkaline phosphatase is biotinylated there is a resulting loss of about 70% of the enzymatic activity of the enzyme, alkaline phosphatase. Substantially the same would be true with respect to the conventional derivatization or modification or biotinylation of other enzymes, such as acid phosphatase and glycosidases. It would appear that in the biotinylation of the enzymes, e.g. alkaline phosphatase, with activated biotin (N-hydroxysuccinimide-biotin), the biotinylation occurs through the epsilon amino group of the lysine residue at or near the site of the enzyme's reactive site. It appears likely, also, that the incorporation of biotin disrupts the enzyme's tertiary structure which is important for full enzyme activity. As a result the direct modification or derivatization, such as biotinylation, of enzymes, such as alkaline phosphatase, has not yielded a satisfactory derivatized enzyme, such as a biotinylated alkaline phosphatase, useful for diagnostic purposes or other purposes.

It is an object of this invention to provide an improved method for the modification or derivatization of functional or biologically active compounds, such as the biotinylation of enzymes, e.g. alkaline phosphatase, acid phosphatase, and other enzymes or biologically functional or active compounds capable of biotinylation or other or similar modification or derivatization.

It is another object of this invention to provide a modified or derivatized active or functional compound, such as a biotinylated alkaline phosphatase or acid phosphatase which is useful per se for diagnostic purposes, such as in connection with the location or identification or determination of other materials.

It is another object of this invention to provide materials and techniques useful in the preparation of compounds which readily attach themselves to or which are readily bound to biotin or avidin moieties or other biologically active or functional compounds or complexes.

In one special embodiment of the practices of this invention there is provided enzyme-based or linked materials and techniques for the preparation of such materials and for the utilization of such materials as diagnostic probes and the like.

How these and other objects of this invention are achieved will become apparent in the light of the accompanying disclosure. In at least one embodiment of the practices of this invention at least one of the foregoing objects will be achieved.

An improved method or technique for the modification or derivatization of functional compounds or biologically active compounds, such as enzymes, hormones, including neurohormones, lectins, antibodies, antigens, proteins, such as steroid binding proteins, hormone receptors and others, is provided. Complexes, such as complexes containing a biologically active or functional compound or compounds or sites, are prepared by attaching or fixing a first compound, which may be a biologically active or functional compound, to a solid substrate under conditions such that the active site of said first compound is protected, thereupon derivatizing or combining said first compound with a derivatizing or modifying compound while said first compound is attached to said solid substrate. The resulting complex or complexed first compound is then released from attachment to said solid substrate to yield the complex, said complex comprising said derivatizing compound attached to said first compound, said first compound, upon release from the solid substrate having available its functional or biologically active site. For example, alkaline phosphatase is attached to a solid substrate, such as dextran beads, preferably through a ligand, such as a competitive inhibitor for alkaline phosphatase, p-aminobenzyl phosphonic acid (p-ABPA). The dextran beads would be first treated with p-ABPA and the alkaline phosphatase brought into contact with the thus-treated dextran beads. The competitive inhibitor p-ABPA serves as a ligand for attachment of the alkaline phosphatase to the dextran beads while at the same time occupying and protecting the active site of the alkaline phosphatase. Thereupon, the thus-attached alkaline phosphatase is derivatized or modified, such as by biotinylation. Upon biotinylation of the alkaline phosphatase, the resulting biotinylated alkaline phosphatase is eluted or removed from attachment to the p-ABPA treated solid substrate or dextran beads to which it was fixed, yielding a biotinylated alkaline phosphatase with the active of functional site of the alkaline phosphatase intact. The complex, while still attached to the dextran beads, can be further treated with a second compound or component which might also, if desired, provide an active or functional site, such as by contact with avidin, which would bind to the modifying or derivatizing moiety, i.e. biotin, of the biotinylated alkaline phosphatase and the thus-produced avidin-biotinylated alkaline phosphatase complex then eluted. This complex, such as a complex comprising avidin-biotinylated alkaline phosphatase, could be employed as a detector in a detection system for the identification and/or location of another compound, such as a biotinylated compound, e.g. a biotinylated nucleotide or biotinylated DNA.

More specifically and with emphasis of the practices of this invention as applied to the biotinylation of enzymes, such as alkaline phosphatase, an improved method or technique for the biotinylation of enzymes, such as alkaline phosphatase, is provided by fixing a competitive inhibitor of the enzyme to a solid substrate, preferably a particle-form substrate. Thereupon, the enzyme is brought into contact with the thus-fixed competitive inhibitor such that the inhibitor interacts with or effectively occupies the enzyme active site. The occupation of the enzyme active site by the inhibitor serves to protect the enzyme, i.e. the enzyme active site, during the subsequent biotinylation of the enzyme. With the active site of the enzyme thus-occupied, the enzyme, now attached to the inhibitor which is fixed to a substrate, is biotinylated, such as by contact or reaction with activated biotin, N-hydroxy-succinimide-biotin. In this operation the enzyme is biotinylated or provided with biotin or biotin moieties attached to the enzyme.

Thereupon, after biotinylation of the enzyme, the resulting biotinylated enzyme is freed from the inhibitor which remains fixed to the substrate, with the resulting production and recovery of a biotinylated enzyme with its reactive site substantially unaffected. This product, the biotinylated enzyme, such as biotinylated alkaline phosphatase, is generally useful in many chemical and/or diagnostic procedures.

The practices of this invention are broadly applicable to enzymes, particularly enzymes which are capable of being biotinylated, such as enzymes which react with activated biotin, N-hydroxy-succinimide-biotin. Suitable such enzymes include not only alkaline phosphatase and acid phosphatase but also other enzymes including glycosidases, e.g. $\beta$ galactosidase and glucose oxidase.

As indicated hereinabove, the biotinylation of the enzymes is readily effected by contacting or reacting the enzyme with activated biotin, N-hydroxy-succinimide-biotin. Other techniques, however, are useful for effecting the biotinylation of the enzymes and all such techniques which produce biotinylated enzymes are useful in the practices of this invention.

-6-

In the practices of this invention, the enzyme to be biotinylated is first brought into contact with a competitive inhibitor so as to temporarily and/or protectively occupy the enzyme reactive or active site during the subsequent biotinylation operation. The inhibitor prior to being brought into contact with the enzyme is first desirably fixed, attached or bound to a substrate. As indicated, the substrate may be a fixed solid substrate or a particle-form solid substrate. The solid substrate may comprise a solid material presenting or providing a glass surface or coating, a polyacrylamide surface or coating or derivatized glass surface or coating, a dextran gel (sepharose) surface or coating or a polystyrene surface or coating. Particularly preferred in the practices of this invention would be a particle-form solid material, such as beads, e.g. sepharose beads, providing or presenting such surfaces. The fixing of the inhibitors to such surfaces substantially readily takes place when the inhibitor is brought into contact with such surfaces.

With respect to the inhibitor employed to protect the enzyme active or reactive site during the subsequent bitoinylation operation, any of the known competitive inhibitors for the enzyme are usefully employed. In the case of alkaline phosphatase, such inhibitors would include the diazonium salts of 1-amino-anthraquinone, 4-benzolyamino-2:5-dimethoxy-aniline, p-nitroaniline, 4-chloro-2-nitroaniline, 2:5-dichloroaniline, o-dianisidine, 4-chloro-o-anisidine, 5-nitro-o-anisidine, 5-chloro-o-toluidine, 4-nitro-o-anisidine, 3-nitro-p-toluidine, 3-nitro-p-anisidine, 4-amino-3:1'-dimethyl azobenzene, 4-amino-2:5-dimethoxy-4-nitroazobenzene, 4-amino-4'-methoxy diphenylamine, 3-nitro-o-toluidine, 4-amino-4'-nitro-3:6-dimethoxy-azobenzene, 4-amino-3':1'-dimethyl-azobenzene, 2-methoxy-5-diethylamino-sulphaniline, 2-benzoylamino-4-methoxy-toluidine, 4-amino-diphenylamine, p-p'-diaminodiphenylamine. With respect to the inhibitor for other enzymes, such as acid phosphatase, such inhibitors include the phosphonic and arsonic acids, such as benzylphosphonic acid, phenylphosphonic acid, 2-phenylethylphosphonic acid, ethylphosphonic acid, p-nitrobenzylphosphonic acid, p-chloro-benzylphosphonic acid, p-aminobenzylphosphonic acid p-aminophenyl- phosphonic acid, cyclohexylmethylphosphonic acid, phenylarsonic acid, p-aminophenylarsonic acid and phosphoric acid. Other inhibitors useful in connection with other enzymes, such as the glycosidases and glucose oxidase, include the inhibitors p-aminophenyl-beta-D-thiogalacto-pyranoside, p-aminophenyl-beta-D-thioglucosopyranoside, 3-aminosuccinyl-3'-aminodipropylamine-beta-D-thiogalacto-pyranoside, p-aminopropylamine-beta-D-thiogalactopyranoside, p-aminobutyl-beta-D-thiogalacto-side, p-aminobutyl-beta-D-thioglucosopyranoside.

Other useful inhibitors and information relative thereto are disclosed in Biochemistry, 1978, 17, No. 5, pp. 915-919; Journal of Clinical Pathology, 1978, 31, pp. 454-460; and Biochimia et Biophysica Acta, 1979, 569, pp. 159-176.

After the enzyme has been protected by having its active or reactive site occupied by the inhibitor which is fixed to a suitable substrate and the enzyme, thus-protected, is biotinylated, such as by contact with activated biotin, e.g. N-hydroxy-succinimide-biotin (NHS-biotin), the biotinylation operation being carried out at a pH in the range abouy 6-7, the resulting biotinylated enzyme is then released or separated from the inhibitor. The release or separation of the biotinylated enzyme from attachment to the fixed inhibitor is readily carried out, such as by increasing the pH to about 9, in buffer containing increased concentrated or phosphate ions which compete for the affinity of the benzyl phosphonic acid for the enzyme.

By way of a specific example of the practices of this invention, an aqueous alkaline phosphatase solution is brought into contact with dextran gel beads, sepharose to which is attached or fixed a competitor inhibitor of alkaline phosphatase inhibitor activity, e.g. p-aminobenzyl phosphonic acid (p-ABPA). Upon contact of the alkaline phosphatase with the fixed p-ABPA, the benzylphosphonic moiety of the p-ABPA fixed to the sepharose beads interacts or occupies the enzyme active site protecting it from subsequent substitution, i.e. the subsequent biotinylation. With the reactive site of the enzyme alkaline phosphatase thus-occupied or attached to the sepharose-fixed p-ABPA, an aqueous solution of activated biotin, N-hydroxy-succinimide-biotin is brought into contact with the enzyme, such as at a pH of 6-7. Under these conditions the biotin will bind or attach to the enzyme, alkaline phosphatase, such as to the amino groups or protein portion thereof. Thereupon, the resulting biotinylated enzyme is removed from attachment to the fixed inhibitor, p-ABPA, by washing or eluting the biotinylated enzyme therefrom by increasing the pH to about 9 and employing an increasingly concentrated solution of phosphate ions.

The resulting eluted biotinylated enzyme, such as the biotinylated alkaline phosphatase, after separation and recovery from the eluate can be used as a probe since it has associated therewith or available thereon the reactive biotin sites.

One special technique and application of the biotinylated enzyme in accordance with this invention would be to employ the biotinylated enzyme for the recovery of avidin and/or streptavidin from mixtures or relatively impure materials containing avidin or streptavidin. Avidin and streptavidin bind strongly to biotin. In this aspect of the practices of this invention, the biotinylated enzyme, such as the biotinylated alkaline phosphatase would not be eluted or detached or freed from the fixed inhibitor but rather the biotinylated enzyme, while still attached to the fixed inhibitor, would be brought into contact with a mixture containing avidin or streptavidin. Because of the strong binding affinity between biotin and avidin or streptavidin, when a material containing avidin or streptavidin is brought into contact with the effectively fixed biotinylated enzyme, the avidin or streptavidin would react with or be bound to the effectively fixed biotin with the result that the avidin or streptavidin would be removed or stripped from the materials containing the same. Thereupon, the resulting biotinylated enzyme, now having avidin or streptavidin attached to the biotin moieties thereof, would be eluted or removed from attachment to the fixed inhibitor and there would be recovered an enzyme complex comprising the enzyme, e.g. alkaline phosphatase, which has been biotinylated and which now has in addition avidin or streptavidin attached or bound to the biotin moieties of the biotinylated enzyme. This technique provides, as indicated, a ready means for the purification and recovery of avidin or streptavidin from impure materials containing the same. Further, this technique also provides a means to provide an enzyme complex as described hereinabove comprising an enzyme, biotin attached thereto and avidin and streptavidin attached to the biotin moieties already fixed to the enzyme.

This complex enzyme-biotin-avidin or streptavidin would be especially useful as a probe to search out, to identify, to determine or locate biotinylated substrates, such as biotinylated DNA, biotinylated peptides or proteins, biotinylated antigens and/or biotinylated antibodies. In this application the location or presence of the biotinylated substrate, such as biotinylated DNA, would be determined by bringing the biotinylated substrate into contact with the enzyme complex comprising the enzyme, e.g. alkaline phosphatase, having attached thereto biotin which, in turn, has attached thereto biotin moieties fixed to the enzyme, avidin or streptavidin. Since avidin or streptavidin has strong binding affinity to biotin, the enzyme complex upon exposure to or contact with the biotinylated substrate becomes readily fixed thereto with the resulting that there would result in the enzyme complex enzyme-biotin-avidin or streptavidin fixed to the biotin moieties of the biotinylated substrate, e.g. biotinylated DNA. The location or presence or determination of the biotinylated DNA substrate would then be evidenced by or elicited by appropriate chemical reaction or physical manifestation initiated or elicited from the reactive site of the enzyme which remains available.

By way of further illustration and explanation of the practices of this invention, and still with emphasis on the applicability of the practices of this invention to derivatization of enzymes, particularly enzymes such as alkaline phosphatase, acid phosphatase and hormone receptor peroxidase, there are set forth hereinbelow examples of the practices of this invention directed to the synthesis of biologically active or functional complexes, specifically complexes comprising alkaline phosphatase-biotin-streptavidin.

### Example No. 1

Preparation of Alkaline Phosphatase-
Biotin-Streptavidin Complex

In the preparation of alkaline phosphatase-biotin-streptavidin complex there are usefully employed as reagents in connection therewith, alkaline phosphatase, immobilized p-aminobenzyl phosphonic acid (p-ABPA) on cross-linked 6% beaded agarose having a capacity of 0.7 mg alkaline phosphatase per ml gel matrix, the biotin compound NHS-$C_7$-biotin, a molecular weight of about 471, in a 0.005 M anhydrous dimethyl sulfoxide solution thereof, streptavidin at a concentration of about 10.2 mg/ml protein, 0.01 M Tris- HCl at a pH of 8.0 and 0.01 M $Na_2$ $HPO_4$ in 0.01 M Tris- HCl at a pH of 8.0.

In the preparation of the complex employing these reagents, 2 ml of a 50% slurry of a Sepharose-p-amino-benzyl phosphonic acid (Seph-p-ABPA) were equilibrated with 10 volumes of 0.01 M Tris-HCl at a pH of 8.0. A solution having a concentration of 1 mg/1 ml alkaline phosphatase and a solution of streptavidin at a concentration of 10 mg/ml were dialyzed against 0.01 M Tris-HCl at a pH of 8.0 at 4°C. for 24 hours. In this operation the buffer volume was changed three times.

One ml of dialyzed alkaline phosphatase was added to 4 ml of a 25% slurry of Seph-p-ABPA and blended or mixed well in a rotator for about 30 minutes at room temperature. Thereupon, the gel was introduced into a small column and the flow-through collected as free enzyme which could be recycled. The gel was then washed with 10 volumes of cold 0.10 M Tris-HCl buffer at a pH of 8.0. The gel was then resuspended in 1 ml of 0.01 Tris-HCl buffer to which was added 0.6 ml of anhydrous diemthyl sulfoxide, drop by drop, with continuous vortating at a slow speed. Subsequently, in the same manner 178 microliters of 0.005 M NHS-C$_7$-biotin was added and the resulting mixture incubated at room temperature for 1.5 hour in the rotator.

At the end of the incubation period there were added 45 ml of cold 0.01 M Tris-HCl at a pH of 8.0 and the resulting admixture mixed well. After letting sit for about 30 minutes at 4°C., the supernatant was removed. This washing operation was repeated two more times. The resulting treated and washed gel was removed and resuspended in 1 ml buffer of 0.01 M Tris-HCl at a pH of 8.0 to which was added 1 ml of dialyzed streptavidin, followed by mixing and incubation at 37°C. for 30 minutes. At the end of the incubation period the gel was poured into a small column and the effluent collected therefrom as free streptavidin which, if desired, could be recycled.

The gel in the column was then washed with 10 volumes of 0.01 Tris-HCl at a pH of 8.0 and the resulting alkaline phosphatase-biotin-streptavidin complex was eluted from the column with 0.01 M $Na_2HPO_4$ in 0.01 M Tris-HCL buffer at a pH of 8.0. In this operation 5 drop fractions were collected. Each fraction was assayed for alkaline phosphatase activity by taking 10 ul of each fraction, 40 ul of buffer and 200 ul of p-nitro-phenyl phosphate (1 mg/ml) in 10% diethanolamine buffer at a pH of 9.0 and incubated at room temperature for about 10 minutes, the resulting solution was read at OD405 nm. The fractions showing enzymatic activity were pooled and applied to nitrocellulose paper dot blotted with decreasing amounts of biotinylated Cytochrome C. The thus-prepared complex was capable of detecting 100 fmole of biotin using NBT-BCIP substrate and 1 fmol using flavone-3-diphosphate, a fluorescence yielding substrate.

This example illustrates not only the preparation of a biologically active or functional compound in accordance with the practices of this invention but also is indicative of the almost exquisite sensitivity and practical utility of the complexes so prepared.

Example No. 2

Preparation of Alkaline Phosphatase-Biotin Streptavidin-Biotin-Antibody Complex

There was prepared a complex comprising alkaline phosphatase-biotin-streptavidin in the manner set forth in Example No. 1 herein. A column containing the complex was washed with 10 M Tris-HCl at a pH of 8.0 and thereupon 0.05 ml of biotinylated (B7)-goat anti-mouse antibody IgG at a concentration of 3 mg/ml in 10 mM Tris-HCl pH 8.0 was added to the column slurry and incubated for 1 hour at $37^O$C. with a gentle rocking motion. Thereupon, the complex was then washed extensively with 10 mM Tris-HCl buffer pH 8.0 and placed back into the column. The column contents were then eluted with 10 M $NaHPO_4$ in 10 mM Tris-HCl at a pH of 8.0. Fractions of the eluate were recovered and tested as follows:

Mouse IgG was spotted onto nitrocellulose paper and air dried and blocked with 0.5% Tween 20 surfactant in PBS for 30 minutes at $37^O$C.. Thereupon, the prepared complex diluted in 1% BSA-0.1% Tween 20 surfactant in PBS was added and incubated for about 1 hour at $37^O$C. with a gentle rocking motion, followed by washing three times with 0.1% Tween 20 surfactant in 1% BSA in PBS for 5 minutes each washing time, followed by washing with 10 mM Tris-HCl. To the resulting prepared test nitrocellulose paper there were added the substrate NBT-BCIP-veronal and incubated at $37^O$C. the spotted mouse IgG developed and detected by the thus-prepared complex.

As indicated hereinabove, the practices of this invention has been emphasized with respect to the preparation of complexes in accordance with this invention wherein the complex comprises an enzyme, such as a biotinylated enzyme, e.g. biotinylated alkaline phosphatase. However, as stated hereinabove, the practices of this invention are applicable to other enzymes, such as, for example, acid phosphatase, glycosidases, or any enzyme in which a competitive enzyme inhibitor may be immobilized on a solid matrix. Further, although it is preferred that the modifying or derivatizing component or compound be biotin, the derivatizing or modifying compound could

-15-

also be a compound or component which provides a glycosyl group to the compound to be derivatized or modified. For example, in the practices of this invention the compounds in accordance with this invention, which could be modified by attachment thereto of a glycosyl group or other sugar moiety. The thus-modified or derivatized compound would then be reacted with a lectin, e.g. Concanavalin A which would attach itself to the glycosyl group, such as in the manner avidin attaches itself to the biotin group or biotin moiety of a biotinylated compound.

As is apparent from the accompanying disclosure in addition to enzymes, other biologically active of functional compounds, such as hormones, including neurohormones, hormone receptors, lectins, antigens, antibodies, proteins, are modified in the manner described herein and then employed in the make-up of biologically active or functional complexes which would have a wide variety of uses. For example, complexes suitably prepared in accordance with the practices of this invention could include complexes which would have at one terminal location an antibody or antigen which would search out and fix to its corresponding antigen or antibody, a hormone which would search out and become attach to its corresponding hormone receptor, a lectin which would search out and attach to a glycosylated compound. The other terminal location of the complex could include a suitable signalling moiety, such as an enzyme, which would be effective in the presence of a suitable substrate, to give a color signal or other chemical or physical change or fluoresce, thereby signalling the attachment of the other end of the complex to a target. For example, when the other end of the complex is a hormone, the complex would be employed to search out or probe for the corresponding hormone receptor, such as in tissue containing the same. With the hormone end of the complex attached to the hormone receptor, the enzyme provided at the other end of the complex would be used, as indicated hereinabove, to signal the location and/or attachment of the hormone terminal portion of the complex to the hormone receptor. In similar manner, the complexes could be used to search out other targets of interest, such as antibodies, antigens, steroids, glycosylated organic molecules, biotinylated molecules or substrates, etc. as described in detail hereinabove.

CLAIMS

1.  A method of preparing a complex of a first compound
    which comprises attaching or fixing said first
    compound to a solid substrate under  conditions
    such that the active site of said first compound
    is protected, derivatizing or combining said
    first compound with a derivatizing compound while
    the active site. of said first compound is attached
    to and/or protected by being fixed to said solid
    substrate, releasing the resulting complexed said
    first compound from attachment to said solid
    substrate to produce said complex, said complex
    comprising said derivatizing compound attached to
    said first compound, said first compound, upon
    release from said solid substrate, having available its active site.

2.  A method of preparing a complex of a first compound
    which comprises attaching or fixing said first
    compound to a solid substrate under conditions
    such that the active site of said first compound
    is protected, derivatizing or combining said
    first compound with a derivatizing compound while
    the active site of said first compound is attached
    to and/or protected by being fixed to said solid
    substrate, attaching a second compound to the
    resulting derivatized or combined first compound,
    said second compound having binding activity
    specific for the derivatizing compound employed
    to derivatize said first compound, releasing the
    resulting complexed said first compound from
    attachment to said solid substrate to produce
    said complex, said complex comprising said second compound attached to said derivatizing compound and said derivatizing compound attached to said first compound, said first compound, upon release from said solid substrate, having available its active. site.

3.  A method in accordance with Claim 1 or Claim 2 wherein said first compound is an enzyme, a hormone, a hormone receptor, a carrier or steroid binding protein, a lectin, an antibody, an antigen or a protein.

4.  A method in accordance with Claim 3 wherein said first compound is alkaline phosphatase, a glycosidase or acid phosphatase.

5.  A method in accordance with Claim 1 or Claim 2 wherein said derivatizing compound is a biotin.

6.  A method in accordance with Claim 1 wherein said first compound is a glycosidase and said derivatizing compound is a biotin, the resulting complex being a biotinylated glycosidase.

7.  A method in accordance with Claim 2 wherein said second compound is avidin or streptavidin.

8.  A method in accordance with Claim 1 wherein said solid substrate is a sepharose substrate, said first compound is a glycosidase and said derivatizing compound is a biotin, the resulting complex being a biotinylated glycosidase.

9.  A method in accordance with Claim 2 wherein said first compound is glycosidase, said derivatizing compound is biotin and said second compound is streptavidin, the resulting complex being streptavidin-biotinylated glycosidase.

10. A method in accordance with Claim 1 or Claim 2 wherein said solid substrate is a glass substrate, a derivatized or modified glass substrate, a polystyrene substrate, a polyacrylamide substrate or a dextran gel or sepharose substrate.

11. A method in accordance with Claim 1 or Claim 2 wherein said substrate is pretreated with a competitive inhibitor for said first compound before attaching said first compound to said solid substrate through said competitive inhibitor.

12. A method in accordance with Claim 11 wherein said competitive inhibitor is p-aminobenzyl phosphonic acid, said substrate is a sepharose substrate, said first compound is alkaline phosphatase, said derivatizing compound is biotin and - in case of the method in accordance with Claim 2 - said second compound is streptavidin or avidin.

13. A method in accordance with Claim 1 wherein said complex, while attached to said solid substrate, is contacted or reacted with a stream or mixture containing a second compound, said second compound having binding activity specific for the derivatizing compound employed to derivatize said first compound so as to extract or remove said second compound from said stream or mixture containing the same.

14. A complex comprising a first compound providing
an active or functional site, said first compound
being derivatized or combined with a derivatizing
compound and a second compound having binding
activity specific for said derivatizing compound
attached to said first compound.

15. A complex in accordance with Claim 14 wherein said
first compound is an enzyme, a hormone, a hormone
receptor, a carrier or steroid binding protein, a lectin, an antibody, an antigen or a protein.

16. A complex in accordance with Claim 15 wherein said
first compound is alkaline phosphatase, a glycosidase
or  acid phosphatase.

17. A complex in accordance with Claim 14 wherein
said derivatizing compound is biotin and said
second compound is avidin or streptavidin.

18. A method of detecting a chemically labeled
nucleotide which comprises bringing said
chemically labeled nucleotide into contact with a
complex comprising a first compound providing an
active or functional site, a derivatizing
compound attached to said first compound of said
complex and a second compound having binding
activity specific for said derivatizing compound
and attached thereto, said second compound also
possessing binding activity for the chemical
moiety employed to chemically label said
chemically labeled nucleotide whereby said
complex becomes attached to the chemical labeling
moiety attached to said nucleotide and eliciting
the presence of said chemically labeled nucleotide,
now attached to said second compound of said complex, by bringing the resulting combination of said complex attached to the chemically nucleotide into contact with a substrate under conditions such that the active or functional site of said first compound making up said complex is effective to produce an observable change in said substrate.

19. A method in accordance with Claim 18 wherein said nucleotide is a deoxyribonucleotide or a ribonucleotide.

20. A method in accordance with Claim 19 wherein said chemically labeled nucleotide is a deoxyribonucleotide and wherein said deoxyribonucleotide comprises a component of a polydeoxyribonucleotide or a DNA molecule.

21. A method in accordance with Claim 19 wherein said chemically labeled nucleotide is a ribonucleotide and wherein said ribonucleotide comprises or is a component of a polyribonucleotide or RNA molecule.

22. A method of detecting a chemically labeled nucleotide which comprises bringing said chemically labeled nucleotide into contact with a complex providing a first compound, a derivatizing compound attached to said first compound of said complex and a second compound having binding activity specific for said derivatizing compound and attached thereto, said second compound also possessing binding activity for the chemical moiety employed to chemically label said chemically labeled nucleotide whereby said complex becomes attached to the chemical labeling moiety of said nucleotide, said first compound being capable of fluorescing and exposing the resulting combination of said chemically labeled nucleotide attached to said complex to radiation to induce fluorescence from said first compound comprising said complex so as to indicate or elicit the presence of said chemically labeled nucleotide attached to said complex.

23. A method in accordance with Claim 18 wherein said chemically labeled nucleotide is a biotinylated nucleotide and wherein said complex comprises horseradish peroxidase, alkaline phosphatase or acid phosphatase as said first compound, biotin as said derivatizing compound and streptavidin or avidin as said second compound.

24. A method of detecting a chemically labeled nucleotide which comprises bringing said chemically labeled nucleotide into contact with a complex providing a first compound, a second compound having binding activity specific for said first compound, said second compound being fluoresceinated, said first compound also possessing specific binding activity with respect to the chemical moiety employed to chemically label said chemically labeled nucleotide wherein said complex becomes attached to the chemical labeling moiety of said nucleotide through said first compound and exposing the resulting combination of said chemically labeled nucleotide attached to said first compound of said complex to radiation whereby said second compound attached to said first compound of said complex is induced to fluoresce so as to indicate or elicit the presence of said chemically labeled nucleotide attached to said complex.

25. A method in accordance with Claim 24 wherein said chemically labeled nucleotide is a biotinylated nucleotide wherein said first compound is goat anti-biotin antibody and wherein said second compound is fluoresceinated rabbit anti-goat antibody.

26. A method in accordance with Claim 18 wherein said chemically labeled nucleotide is a glycosylated nucleotide and wherein said complex comprises as said first compound a lectin.

27 A method in accordance with Claim 26 wherein said lectin is Concanavalin A.

28. A method of detecting a hormone which comprises bringing material containing said hormone into contact with a complex under conditions to bring the complex into contact with said hormone, said complex comprising a hormone receptor providing an active or functional site for attachment to said hormone when in contact therewith, a derivatizing compound attached to said hormone receptor of said complex and a second compound attached to said derivatizing compound, said second compound providing an active or functional site such that when said complex bound to said hormone is brought into contact with a substrate said complex is capable of producing an observable change in said substrate, bringing said complex into contact with said hormone to attach said complex to said hormone via said hormone receptor and bringing the resulting combined hormone-complex into contact with said substrate to effect an observable change in said substrate thereby eliciting the presence of said hormone in said material.

29.   A method of detecting an antibody which comprises bringing material containing said antibody into contact with a complex under conditions to bring said complex into contact with said antibody, said complex comprising an antigen for said antibody for attachment to said antibody when in contact therewith, a derivatizing compound attached to said antigen of said complex and a second compound attached to said derivatizing compound, said second compound providing an active or functional site such that when said complex bound to said antibody is brought into contact with a substrate, said complex is capable of producing an observable change in said substrate, bringing said complex into contact with said antibody to attach said complex to said antibody via said antigen and bringing the resulting combined antibody-complex into contact with said substrate to effect an observable change in said substrate, thereby eliciting the presence of said antibody in said material.

30.     A method of detecting an antigen which comprises bringing material containing said antigen into contact with a complex under conditions to bring said complex comprising an antibody for said antigen for attachment to said antigen when in contact therewith, a derivatizing compound attached to said antibody of said complex and a second compound attached to said derivatizing compound, said second compound providing an active or functional site such that when said complex bound to said antigen is brought into contact with a substrate, said complex is capable of producing an observable change in said substrate, bringing said complex into contact with said antigen to attach said complex to said antigen via said antibody and bringing the resulting combined antigen-complex into contact with said substrate to effect an observable change in said substrate, thereby eliciting the presence of said antigen in said material.